# EUROPÄISCHE PATENTSCHRIFT

(11) **EP 3 233 781 B1**
(45) Veröffentlichungstag und Bekanntmachung des Hinweises auf die Patenterteilung: **28.08.2019**
(21) Anmeldenummer: 15807656.2
(22) Anmeldetag: 08.12.2015
(51) Int. Cl.: C07C 49/84

(54) **PHOTOLABILE DUFTSTOFFVORLÄUFERVERBINDUNG**
PHOTOLABILE FRAGRANCE PRECURSOR COMPOUND
COMPOSÉ PRÉCURSEUR DE PARFUM PHOTO-LABILE

(30) Priorität: 17.12.2014 DE 102014226196
(43) Veröffentlichungstag der Anmeldung: 25.10.2017
(73) Patentinhaber: Henkel AG & Co. KGaA, 40589 Düsseldorf (DE)
(72) Erfinder: KROPF, Christian, 40724 Hilden (DE); HUCHEL, Ursula, 50733 Köln (DE); BLUHM, Nadine, 40233 Düsseldorf (DE); BAUER, Andreas, 41564 Kaarst (DE)
(86) Internationale Anmeldenummer: PCT/EP2015/078982
(87) Internationale Veröffentlichungsnummer: WO 2016/096539

(56) Entgegenhaltungen:
- WO-A1-2007/054446
- WO-A1-2011/101180
- WO-A2-2011/003029
- DE-A1- 2 420 474
- US-A- 4 101 584
- US-A- 4 218 479
- US-A- 4 921 528
- US-A1- 2014 275 282
- US-B2- 6 949 680
- MUTHUSAMY S ET AL: "Indium triflate: a mild and efficient Lewis acid catalyst for O-H insertion reactions of alpha-diazo ketones", TETRAHEDRON LETTERS, PERGAMON, GB, Bd. 43, Nr. 17, 22. April 2002 (2002-04-22), Seiten 3133-3136, XP004347877, ISSN: 0040-4039, DOI: 10.1016/S0040-4039(02)00489-6

## Beschreibung

Die vorliegende Erfindung liegt auf dem Gebiet der Duftspeicherstoffe, wie sie zum Beispiel auf dem Gebiet der Wasch- oder Reinigungsmittel, kosmetischen Mittel sowie Luftpflegemittel Einsatz finden. Die Erfindung betrifft spezielle Phenacylether, die als photolabile Duftspeicherstoffe fungieren. Ferner betrifft die vorliegende Erfindung Wasch- und Reinigungsmittel, kosmetische Mittel sowie Luftpflegemittel, welche solche Phenacylether enthalten. Ferner betrifft sie ein Verfahren zur lang anhaltenden Beduftung von Oberflächen und ebenso ein Verfahren zur lang anhaltenden Raumbeduftung.

Wasch- sowie Reinigungsmittel bzw. kosmetische Mittel enthalten zumeist Duftstoffe, die den Mitteln einen angenehmen Geruch verleihen. Die Duftstoffe maskieren dabei zumeist den Geruch der anderen Inhaltstoffe, so dass beim Verbraucher ein angenehmer Geruchseindruck entsteht.

Duftstoffe sind insbesondere im Bereich Waschmittel wichtige Bestandteile der Formulierung, da die Wäsche sowohl im feuchten als auch im trockenen Zustand einen angenehmen und frischen Duft aufweisen soll. Grundsätzlich bringt der Einsatz von Duftstoffen das Problem mit sich, dass Duftstoffe mehr oder minder leicht flüchtige Verbindungen sind, jedoch ein lange anhaltender Dufteffekt erwünscht ist. Insbesondere bei Riechstoffen, die die frischen und leichten Noten des Parfüms darstellen und infolge ihres verhältnismäßigen hohen Dampfdruck besonders schnell verdampfen, ist die gewünschte Langlebigkeit des Dufteindrucks kaum erreichbar. Photolabile Duftspeicherstoff-Moleküle sind im Stand der Technik bekannt und stellen eine Möglichkeit dar, Duftstoffe verzögert freizusetzen. Durch die Einwirkung des Sonnenlichts oder einer anderen elektromagnetischen Strahlungsquelle bestimmter Wellenlänge wird der Bruch einer kovalenten Bindung im Duftspeicherstoff-Molekül induziert, wodurch ein Duftstoff freigesetzt wird.

So offenbart das US-Patent 6,949,680 beispielsweise die Verwendung bestimmter Phenyl- oder Pyridylketone als photoaktivierbare Substanzen, die in Gegenwart von Licht in einer photochemischen Fragmentierung ein terminales Alken als Aktivstoff freisetzen. Der genannte Aktivstoff besitzt beispielsweise eine duftgebende oder antimikrobielle Aktivität, die durch den photochemisch induzierten Zerfall erst verzögert und über einen längeren Zeitraum hinweg auf einer bestimmten Oberfläche freigesetzt wird.

Die internationale Patentveröffentlichung WO 2009/118219 A1 offenbart photoaktivierbare Substanzen, welche eine Freisetzung von cyclischen Terpenen oder cyclischen Terpenoiden ermöglichen und die WO 2011/101180 offenbart die Verwendung bestimmter Ketone als photoaktivierbare Substanzen, die in Gegenwart von Licht in einer photochemischen Fragmentierung einen Aktivstoff freisetzen. Der genannte Aktivstoff besitzt beispielsweise eine duftgebende Aktivität, die durch den photochemisch induzierten Zerfall erst verzögert und über einen längeren Zeitraum hinweg auf einer bestimmten Oberfläche freigesetzt wird.

Jedoch ist die Duftintensität bei Verwendung bekannter Duftspeicher-Moleküle gering und der Dufteffekt nur von kurzer Dauer. Ferner verursachen herkömmliche Duftspeicher-Moleküle nach der Freisetzung des Duftstoffs eine mit dem bloßen Auge wahrnehmbare Gelbfärbung, was unerwünscht ist. Daher besteht ein Bedarf an Duftspeicher-Molekülen, die Duftstoffe effektiv freisetzen und sich nicht verfärben.

Es wurde nun überraschenderweise gefunden, dass spezielle Phenacylether der Formel (II) bis (XXV) geeignet sind, über einen langen Zeitraum Duftstoffe freizusetzen ohne sich dabei zu verfärben. Dies führt zu einem optisch ansprechenderen Erscheinungsbild des Gegenstands, wie beispielsweise Textilien, auf den die erfindungsgemäße Verbindung aufgebracht wurde. Ferner setzen die Verbindungen gemäß der Erfindung über einen langen Zeitraum Duftstoffe frei und sorgen somit für einen lang anhaltenden Dufteffekt, insbesondere im Zusammenhang mit der Textilbehandlung. Zum Beispiel konnte bei der Anwendung der hierin beschriebenen Phenacylether in einem Wäschebehandlungsmittels, d.h. einem Waschmittel oder Weichspüler, eine verbesserte Langzeitduftwirkung der behandelten Wäsche erzielt werden. Die erfindungsgemäßen Verbindungen ermöglichen es zudem, die Gesamtmenge an Duftstoff, welche im Mittel enthalten ist, zu reduzieren, und dennoch Geruchsvorteile auf den gewaschenen Textilien zu erzielen, insbesondere mit Blick auf das Frischeempfinden.

In einem ersten Aspekt richtet sich die vorliegende Erfindung daher auf Verbindungen der Formeln (II) bis (XXV) wie in Anspruch 1 definiert.

In einem weiteren Aspekt betrifft die vorliegende Erfindung ein Wasch- oder Reinigungsmittel, enthaltend mindestens eine Verbindung der Formeln (II) bis (XXV), wie hierin beschrieben.

Ein weiterer Gegenstand der Erfindung ist ein kosmetisches Mittel, das mindestens eine der hierin beschriebenen Verbindungen der Formeln (II) bis (XXV) umfasst, dadurch gekennzeichnet, dass die Verbindung in Mengen zwischen 0,0001 und 50 Gew.-%, bezogen auf das gesamte Mittel, enthalten ist.

Noch ein weiterer Gegenstand der Erfindung ist ein Luftpflegemittel, das mindestens eine der erfindungsgemäßen Verbindungen der Formeln (II) bis (XXV) enthält, dadurch gekennzeichnet, dass die Verbindung in Mengen zwischen 0,0001 und 50 Gew.-%, bezogen auf das gesamte Mittel, enthalten ist.

Schließlich wird ein Verfahren zur lang anhaltenden Beduftung von Oberflächen in denen eine Verbindung wie hierin beschrieben auf die zu beduftende Oberfläche aufgebracht wird und die genannte Oberfläche anschließend einer elektromagnetischen Strahlung, vorzugsweise umfassend die Wellenlängen von 200 bis 600 nm, ausgesetzt wird, offenbart, welches nicht erfindungsgemäß ist.

"Mindestens ein", wie hierin verwendet, bezieht sich auf 1 oder mehr, beispielsweise 2, 3, 4, 5, 6, 7, 8, 9 oder mehr. Im Zusammenhang mit Bestandteilen der hierin beschriebenen Verbindung bezieht sich diese Angabe nicht auf die absolute Menge an Molekülen sondern auf die Art des Bestandteils. "Mindestens eine Verbindung der Formel (I)" bedeutet daher beispielsweise ein oder mehrere verschiedene Verbindungen der Formel (I), d.h. eine oder mehrere verschiedene Arten von Verbindungen der Formel (I). Zusammen mit Mengenangaben beziehen sich die Mengenangaben auf die Gesamtmenge der entsprechend bezeichneten Art von Bestandteil, wie bereits oben definiert.

Die erfindungsgemäße Verbindung sind Verbindungen gemäß einer der folgenden Formeln (II) bis (XXV)

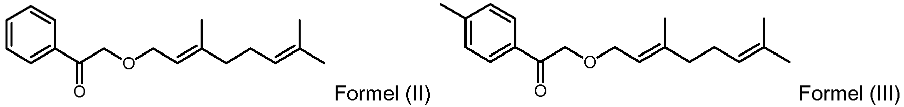

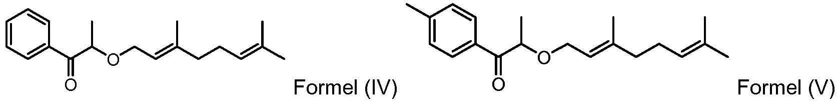

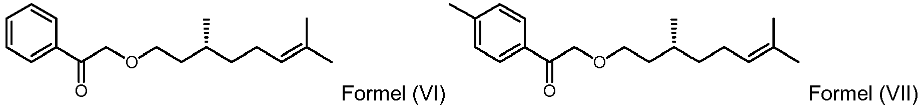

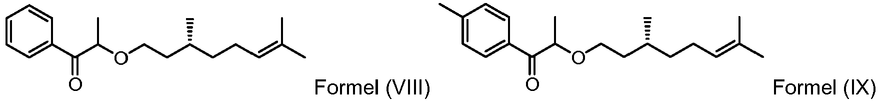

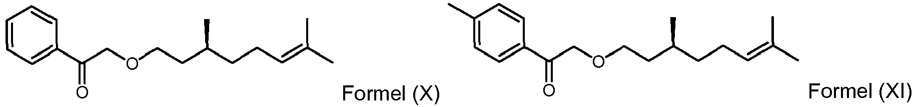

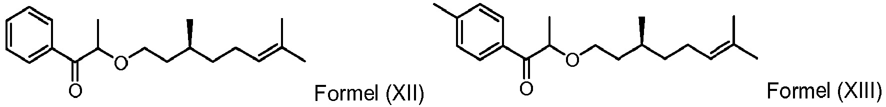

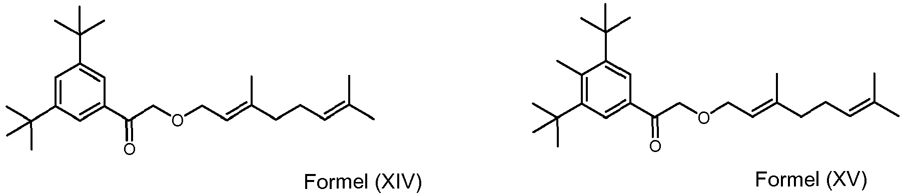

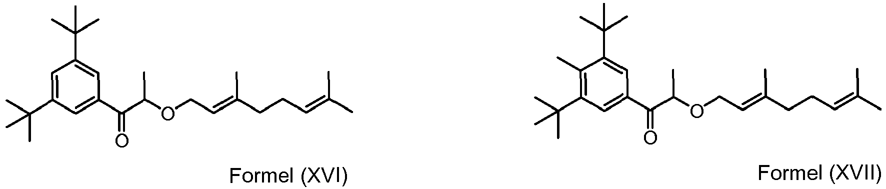

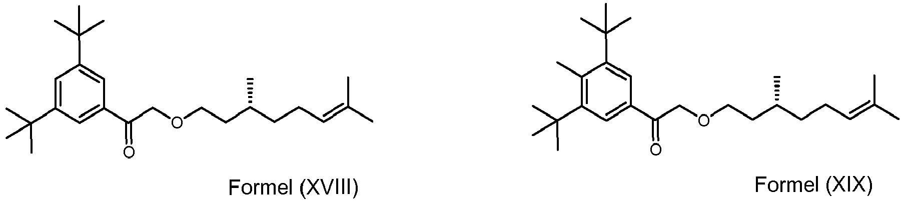

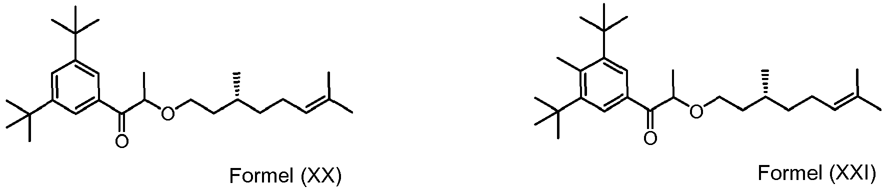

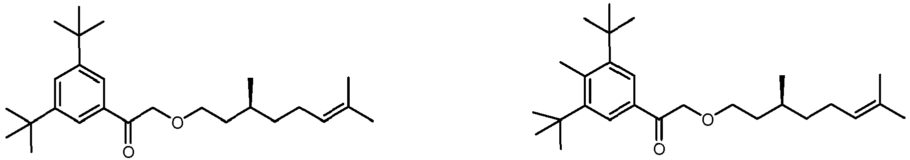

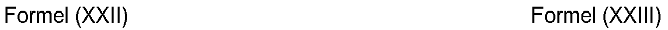

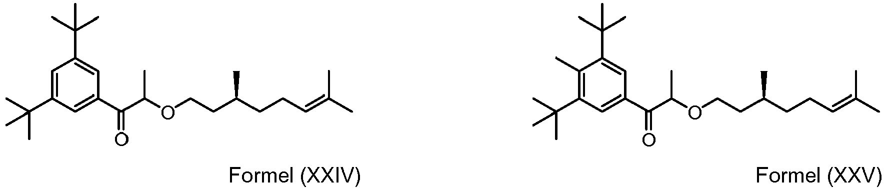

Erfindungsgemäß können die Verbindungen (II) bis (XXV) einzeln aber auch als Mischungen eingesetzt werden. Beispielsweise können auch die jeweiligen racemische Mischungen verwendet werden, wie eine Mischung der Verbindungen (IV) und (VI) oder eine Mischung der Verbindungen (V) und (VII).

Die erfindungsgemäßen Verbindungen lassen sich stabil in die üblichen Wasch- oder Reinigungsmittelmatrices, in Kosmetika und bestehende Riechstoffkompositionen einarbeiten. Sie ermöglichen eine verzögerte Freisetzung der gespeicherten Duftstoffketone und -aldehyde. Bevorzugte Duftstoffe sind Geranial, Neral, (R)-Citronellal und (S)-Citronellal. Diese Duftstoffe verleihen üblichen Wasch- oder Reinigungsmitteln sowie Kosmetika einen besonders lange anhaltenden Frischeeindruck. Insbesondere das getrocknete, gewaschene Textil profitiert von der guten Langzeitfrischeduftwirkung. Die langsame Freisetzung des gespeicherten Riechstoffes erfolgt nach Einwirkung von Licht (elektromagnetische Strahlung) der Wellenlänge 200 bis 600 nm.

Ein weiterer Gegenstand der vorliegenden Erfindung ist ein Wasch- oder Reinigungsmittel, vorzugsweise ein Waschmittel, Weichspüler oder Waschhilfsmittel, enthaltend mindestens eine Verbindung der Formeln (II) bis (XXV), wobei besagte Verbindung vorzugsweise in Mengen zwischen 0,0001 und 5 Gew.-%, vorteilhafterweise zwischen 0,001 und 4 Gew.-%, weiter vorteilhaft zwischen 0,005 und 3 Gew.-%, insbesondere zwischen 0,01 und 2 Gew.-%, jeweils bezogen auf das Gesamtgewicht des Mittels, enthalten ist. Geeignete Reinigungsmittel sind z.B. Reinigungsmittel für harte Oberflächen, insbesondere Geschirrspülmittel. Ebenso kann es sich bei dem Reinigungsmittel beispielsweise um Haushaltsreiniger, Allzweckreiniger, Fensterreiniger, Fußbodenreiniger usw. handeln. In verschiedenen Ausführungsformen kann es sich um ein Produkt zur Reinigung von WC-Becken und Urinalen handeln, insbesondere um einen Spülreiniger zum Einhängen in das WC-Becken.

Gemäß einer bevorzugten Ausführungsform der Erfindung enthält das erfindungsgemäße Wasch- oder Reinigungsmittel mindestens ein Tensid, ausgewählt aus anionischen, kationischen, nichtionischen, zwitterionischen und amphoteren Tensiden oder Mischungen davon.

Gemäß einer weiteren bevorzugten Ausführungsform der Erfindung liegt das erfindungsgemäße Mittel in fester oder flüssiger Form vor.

Ein weiterer Gegenstand der Erfindung ist ein kosmetisches Mittel, enthaltend mindestens eine Verbindung gemäß der Formeln (II) bis (XXV), welches die Verbindung in Mengen zwischen 0,0001 und 50 Gew.-%, vorteilhafterweise zwischen 0,001 und 5 Gew.-%, weiter vorteilhaft zwischen 0,005 und 3 Gew.-%, insbesondere zwischen 0,01 und 2 Gew.-%, jeweils bezogen auf das gesamte Mittel, enthält.

Ein weiterer Gegenstand der Erfindung ist ein Luftpflegemittel (z.B. Raumlufterfrischer, Raumdeodorant, Raumspray usw.), enthaltend mindestens eine Verbindung gemäß der Formeln (II) bis (XXV), wobei die Verbindung der Formeln (II) bis (XXV) in Mengen zwischen 0,0001 und 50 Gew.-%, vorzugsweise zwischen 0,001 und 5 Gew.-%, bevorzugt zwischen 0,01 und 3 Gew.-%, besonders bevorzugt zwischen 0,1 und 2 Gew.-%, jeweils bezogen auf das Gesamtgewicht des Mittels, enthalten ist.

Gemäß einer weiteren bevorzugten Ausführungsform der Erfindung sind in einem erfindungsgemäßen Mittel (d.h. Wasch- oder Reinigungsmittel, kosmetisches Mittel, oder Luftpflegemittel) zusätzliche Duftstoffe enthalten, insbesondere ausgewählt aus der Gruppe umfassend Duftstoffe natürlichen oder synthetischen Ursprungs, bevorzugt leichter flüchtige Duftstoffe, höhersiedende Duftstoffe, feste Duftstoffe und/oder haftfeste Duftstoffe.

Haftfeste Riechstoffe, die im Rahmen der vorliegenden Erfindung mit Vorteil einsetzbar sind, sind beispielsweise etherische Öle wie Angelikawurzelöl, Anisöl, Arnikablütenöl, Basilikumöl, Bayöl, Bergamottöl, Champacablütenöl, Edeltannenöl, Edeltannenzapfenöl, Elemiöl, Eukalyptusöl, Fenchelöl, Fichtennandelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaivabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Neroliöl, Niaouliöl, Olibanumöl, Orangenöl, Origanumöl, Palmarosaöl, Patschuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-Öl, Ysop-Öl, Zimtöl, Zimtblätteröl, Zitronellöl, Zitronenöl sowie Zypressenöl.

Aber auch höhersiedende bzw. feste Riechstoffe natürlichen oder synthetischen Ursprungs können im Rahmen der vorliegenden Erfindung als haftfeste Riechstoffe bzw. Riechstoffgemische, also Duftstoffe eingesetzt werden. Zu diesen Verbindungen zählen die nachfolgend genannten Verbindungen sowie Mischungen aus diesen: Ambrettolid, alpha-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylalkohol, Benzylacetat, Benzylbenzoat, Benzylformiat, Benzylvalerianat, Borneol, Bornylacetat, alpha-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinondimethylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p-Kresol-methylether, Cumarin, p-Methoxyacetophenon, Methyl-n-amylketon, Methylanthranilsäuremethylester, p-Methylacetophenon, Methylchavikol, p-Methylchinolin, Methyl-beta-naphthylketon, Methyl-n-nonylacetaldehyd, Methyl-n-nonylketon, Muskon, beta-Naphtholethylether, beta-Naphtholmethylether, Nerol, Nitrobenzol, n-Nonylaldehyd, Nonylakohol, n-Octylaldehyd, p-Oxy-Acetophenon, Pentadekanolid, beta-Phenylethylalkohol, Phenylacetaldehyd-Dimethylacetal, Phenylessigsäure, Pulegon, Safrol, Salicylsäureisoamylester, Salicylsäuremethylester, Salicylsäurehexylester, Salicylsäurecyclohexylester, Santalol, Skatol, Terpineol, Thymen, Thymol, gamma-Undecalacton, Vanillin, Veratrumaldehyd, Zimtaldehyd, Zimatalkohol, Zimtsäure, Zimtsäureethylester, Zimtsäurebenzylester. Zu den leichter flüchtigen Duftstoffen zählen insbesondere die niedriger siedenden Riechstoffe natürlichen oder synthetischen Ursprung, die allein oder in Mischungen eingesetzt werden können. Beispiele für leichter flüchtige Duftstoffe sind Alkylisothiocyanate (Alkylsenföle), Butandion, Limonen, Linalool, Linaylacetat und -propionat, Menthol, Menthon, Methyl-n-heptenon, Phellandren, Phenylacetaldehyd, Terpinylacetat, Zitral, Zitronellal.

Nach einer weiteren bevorzugten Ausführungsform weist das erfindungsgemäße Mittel (d.h. Wasch- oder Reinigungsmittel, kosmetisches Mittel, oder Luftpflegemittel), wenigstens eine, vorzugsweise mehrere, aktive Komponenten auf, insbesondere wasch-, pflege-, reinigungsaktive und/oder kosmetische Komponenten, vorteilhafterweise ausgewählt aus der Gruppe umfassend anionische Tenside, kationische Tenside, amphotere Tenside, nichtionische Tenside, Acidifizierungsmittel, Alkalisierungsmittel, Anti-Knitter-Verbindungen, antibakterielle Stoffe, Antioxidantien, Antiredepositionsmittel, Antistatika, Buildersubstanzen, Bleichmittel, Bleichaktivatoren, Bleichstabilisatoren, Bleichkatalysatoren, Bügelhilfsmittel, Cobuilder, Duftstoffe, Einlaufverhinderer, Elektrolyte, Enzyme, Farbschutzstoffe, Färbemittel, Farbstoffe, Farbübertragungsinhibitoren, Fluoreszenzmittel, Fungizide, Germizide, geruchskomplexierende Substanzen, Hilfsmittel, Hydrotrope, Klarspüler, Komplexbildner, Konservierungsmittel, Korrosionsinhibitoren, wassermischbare organische Lösungsmittel, optische Aufheller, Parfüme, Parfümträger, Perlglanzgeber, pH-Stellmittel, Phobier- und Imprägniermittel, Polymere, Quell- und Schiebefestmittel, Schauminhibitoren, Schichtsilikate, schmutzabweisende Stoffe, Silberschutzmittel, Silikonöle, Soil-release-Wirkstoffe, UV-Schutz-Substanzen, Viskositätsregulatoren, Verdickungsmittel, Verfärbungsinhibitoren, Vergrauungsinhibitoren, Vitamine und/oder Weichspüler. Im Sinne dieser Erfindung beziehen sich Mengenangaben in Gew.-%, wenn nicht anders angegeben, auf das Gesamtgewicht des erfindungsgemäßen Mittels.

Die Mengen der einzelnen Inhaltsstoffe in den erfindungsgemäßen Mitteln (d.h. Wasch- oder Reinigungsmittel, kosmetisches Mittel oder Luftpflegemittel) orientieren sich jeweils am Einsatzzweck der betreffenden Mittel und der Fachmann ist mit den Größenordnungen der einzusetzenden Mengen der Inhaltsstoffe grundsätzlich vertraut oder kann diese der zugehörigen Fachliteratur entnehmen. Je nach Einsatzzweck der erfindungsgemäßen Mittel wird man beispielsweise den Tensidgehalt höher oder niedriger wählen. Üblicherweise kann z.B. der Tensidgehalt von Waschmitteln zwischen 10 und 50 Gew.-%, vorzugsweise zwischen 12,5 und 30 Gew.-% und insbesondere zwischen 15 und 25 Gew.-% betragen, während z.B. Reinigungsmittel für das maschinelle Geschirrspülen z.B. zwischen 0,1 und 10 Gew.-%, vorzugsweise zwischen 0,5 und 7,5 Gew.-% und insbesondere zwischen 1 und 5 Gew.-% Tenside enthalten können.

Die erfindungsgemäßen Mittel (d.h. Wasch- oder Reinigungsmittel, kosmetisches Mittel oder Luftpflegemittel) können Tenside enthalten, wobei bevorzugt anionische Tenside, nichtionische Tenside und deren Gemische, aber auch kationische Tenside in Frage kommen. Geeignete nichtionische Tenside sind insbesondere Ethoxylierungs- und/oder Propoxylierungsprodukte von Alkylglykosiden und/oder linearen oder verzweigten Alkoholen mit jeweils 12 bis 18 Kohlenstoffatomen im Alkylteil und 3 bis 20, vorzugsweise 4 bis 10 Alkylethergruppen. Weiterhin sind entsprechende Ethoxylierungs- und/oder Propoxylierungsprodukte von N-Alkylaminen, vicinalen Diolen, Fettsäureestern und Fettsäureamiden, die hinsichtlich des Alkylteils den genannten langkettigen Alkoholderivaten entsprechen, sowie von Alkylphenolen mit 5 bis 12 Kohlenstoffatomen im Alkylrest brauchbar.

Geeignete anionische Tenside sind insbesondere Seifen und solche, die Sulfat- oder SulfonatGruppen mit bevorzugt Alkaliionen als Kationen enthalten. Verwendbare Seifen sind bevorzugt die Alkalisalze der gesättigten oder ungesättigten Fettsäuren mit 12 bis 18 Kohlenstoffatomen. Derartige Fettsäuren können auch in nicht vollständig neutralisierter Form eingesetzt werden. Zu den brauchbaren Tensiden des Sulfat-Typs gehören die Salze der Schwefelsäurehalbester von Fettalkoholen mit 12 bis 18 Kohlenstoffatomen und die Sulfatierungsprodukte der genannten nichtionischen Tenside mit niedrigem Ethoxylierungsgrad. Zu den verwendbaren Tensiden vom Sulfonat-Typ gehören lineare Alkylbenzolsulfonate mit 9 bis 14 Kohlenstoffatomen im Alkylteil, Alkansulfonate mit 12 bis 18 Kohlenstoffatomen, sowie Olefinsulfonate mit 12 bis 18 Kohlenstoffatomen, die bei der Umsetzung entsprechender Monoolefine mit Schwefeltrioxid entstehen, sowie alpha-Sulfofettsäureester, die bei der Sulfonierung von Fettsäuremethyl- oder -ethylestern entstehen.

Kationische Tenside werden vorzugsweise unter den Esterquats und/oder den quaternären Ammoniumverbindungen (QAV) gemäß der allgemeinen Formel (R^{I})(R^{II})(R^{III})(R^{IV})N⁺ X⁻ ausgewählt, in der R¹ bis R^{IV} für gleiche oder verschiedene C₁₋₂₂-Alkylreste, C₇₋₂₈-Arylalkylreste oder heterozyklische Reste stehen, wobei zwei oder im Falle einer aromatischen Einbindung wie im Pyridin sogar drei Reste gemeinsam mit dem Stickstoffatom den Heterozyklus, z.B. eine Pyridinium- oder Imidazoliniumverbindung, bilden, und X⁻ für Halogenidionen, Sulfationen, Hydroxidionen oder ähnliche Anionen steht. QAV sind durch Umsetzung tertiärer Amine mit Alkylierungsmitteln, wie z.B. Methylchlorid, Benzylchlorid, Dimethylsulfat, Dodecylbromid, aber auch Ethylenoxid herstellbar. Die Alkylierung von tertiären Aminen mit einem langen Alkyl-Rest und zwei Methyl-Gruppen gelingt besonders leicht, auch die Quaternierung von tertiären Aminen mit zwei langen Resten und einer Methyl-Gruppe kann mit Hilfe von Methylchlorid unter milden Bedingungen durchgeführt werden. Amine, die über drei lange Alkyl-Reste oder Hydroxysubstituierte Alkyl-Reste verfügen, sind wenig reaktiv und werden z.B. mit Dimethylsulfat quaterniert. In Frage kommende QAV sind beispielweise Benzalkoniumchlorid (N Alkyl-N,N dimethyl-benzylammoniumchlorid), Benzalkon B (m,p-Dichlorbenzyldimethyl-C₁₂-alkylammoniumchlorid, Benzoxoniumchlorid (Benzyl-dodecyl-bis-(2-hydroxyethyl)-ammoniumchlorid), Cetrimoniumbromid (N-Hexadecyl-N,N-trimethyl-ammoniumbromid), Benzetoniumchlorid (N,N Dimethyl-N [2-[2-[p-(1,1,3,3-tetramethylbutyl)phenoxy]-ethoxy]-ethyl]-benzyl-ammoniumchlorid), Dialkyldimethylammoniumchloride wie Di-n-decyl-dimethyl-ammoniumchlorid, Didecyldimethylammonium-bromid, Dioctyl-dimethyl-ammoniumchlorid, 1-Cetylpyridiniumchlorid und Thiazoliniodid sowie deren Mischungen. Bevorzugte QAV sind die Benzalkoniumchloride mit C₈-C₂₂-Alkylresten, insbesondere C₁₂-C₁₄-Alkyl-benzyl-dimethylammoniumchlorid.

Bevorzugte Esterquats sind Methyl-N-(2-hydroxyethyl)-N,N-di(talgacyl-oxyethyl)ammonium-metho-sulfat, Bis-(palmitoyl)-ethyl-hydroxyethyl-methyl-ammonium-methosulfat oder Methyl-N,N-bis(acyl-oxyethyl)-N-(2-hydroxyethyl)ammonium-methosulfat. Handelsübliche Beispiele sind die von der Firma Stepan unter dem Warenzeichen Stepantex® vertriebenen Methylhydroxyalkyldialkoyloxyalkylammoniummethosulfate oder die unter dem Handelsnamen Dehyquart bekannten Produkte der Firma BASF SE beziehungsweise die unter der Bezeichnung Rewoquat bekannten Produkte des Herstellers Evonik.

Tenside sind in den erfindungsgemäßen Mitteln (d.h. Wasch- oder Reinigungsmittel, kosmetisches Mittel oder Luftpflegemittel) in Mengenanteilen von vorzugsweise 5 Gew.-% bis 50 Gew.-%, insbesondere von 8 Gew.-% bis 30 Gew.-%, enthalten. Insbesondere in Wäsche-Nachbehandlungsmitteln werden vorzugsweise bis zu 30 Gew.-%, insbesondere 5 Gew.-% bis 15 Gew.-% Tenside, unter diesen bevorzugt wenigstens anteilsweise kationische Tenside, eingesetzt.

Ein erfindungsgemäßes Mittel, insbesondere Wasch- oder Reinigungsmittel, enthält vorzugsweise mindestens einen wasserlöslichen und/oder wasserunlöslichen, organischen und/oder anorganischen Builder. Zu den wasserlöslichen organischen Buildersubstanzen gehören Polycarbonsäuren, insbesondere Citronensäure und Zuckersäuren, monomere und polymere Aminopolycarbonsäuren, insbesondere Methylglycindiessigsäure, Nitrilotriessigsäure und Ethylendiamintetraessigsäure sowie Polyasparaginsäure, Polyphosphonsäuren, insbesondere Aminotris(methylenphosphonsäure), Ethylendiamintetrakis(methylenphosphonsäure) und 1-Hydroxyethan-1,1-diphosphonsäure, polymere Hydroxyverbindungen wie Dextrin sowie polymere (Poly-)carbonsäuren, polymere Acrylsäuren, Methacrylsäuren, Maleinsäuren und Mischpolymere aus diesen, die auch geringe Anteile polymerisierbarer Substanzen ohne Carbonsäurefunktionalität einpolymerisiert enthalten können. Geeignete, wenn auch weniger bevorzugte Verbindungen dieser Klasse sind Copolymere der Acrylsäure oder Methacrylsäure mit Vinylethern, wie Vinylmethylethern, Vinylester, Ethylen, Propylen und Styrol, in denen der Anteil der Säure mindestens 50 Gew.-% beträgt. Die organischen Buildersubstanzen können, insbesondere zur Herstellung flüssiger Mittel, in Form wässriger Lösungen, vorzugsweise in Form 30- bis 50-gewichtsprozentiger wässriger Lösungen eingesetzt werden. Alle genannten Säuren werden in der Regel in Form ihrer wasserlöslichen Salze, insbesondere ihre Alkalisalze, eingesetzt.

Organische Buildersubstanzen können, falls gewünscht, in Mengen bis zu 40 Gew.-%, insbesondere bis zu 25 Gew.-% und vorzugsweise von 1 Gew.-% bis 8 Gew.-% enthalten sein. Mengen nahe der genannten Obergrenze werden vorzugsweise in pastenförmigen oder flüssigen, insbesondere wasserhaltigen, erfindungsgemäßen Mitteln eingesetzt. Erfindungsgemäße Wäschenachbehandlungsmittel, wie z.B. Weichspüler, können gegebenenfalls auch frei von organischem Builder sein.

Als wasserlösliche anorganische Buildermaterialien kommen insbesondere Alkalisilikate und Polyphosphate, vorzugsweise Natriumtriphosphat, in Betracht. Als wasserunlösliche, wasserdispergierbare anorganische Buildermaterialien können insbesondere kristalline oder amorphe Alkalialumosilikate, falls gewünscht, in Mengen von bis zu 50 Gew.-%, vorzugsweise nicht über 40 Gew.-% und in flüssigen Mitteln insbesondere von 1 Gew.-% bis 5 Gew.-%, eingesetzt werden. Unter diesen sind die kristallinen Natriumalumosilikate in Waschmittelqualität, insbesondere Zeolith A, P und gegebenenfalls X, bevorzugt. Mengen nahe der genannten Obergrenze werden vorzugsweise in festen, teilchenförmigen Mitteln eingesetzt. Geeignete Alumosilikate weisen insbesondere keine Teilchen mit einer Korngröße über 30 µm auf und bestehen vorzugsweise zu wenigstens 80 Gew.-% aus Teilchen mit einer Größe unter 10 µm.

Geeignete Substitute beziehungsweise Teilsubstitute für das genannte Alumosilikat sind kristalline Alkalisilikate, die allein oder im Gemisch mit amorphen Silikaten vorliegen können. Die in den erfindungsgemäßen Mitteln als Gerüststoffe brauchbaren Alkalisilikate weisen vorzugsweise ein molares Verhältnis von Alkalioxid zu SiO₂ unter 0,95, insbesondere von 1:1,1 bis 1:12 auf und können amorph oder kristallin vorliegen. Bevorzugte Alkalisilikate sind die Natriumsilikate, insbesondere die amorphen Natriumsilikate, mit einem molaren Verhältnis Na₂O:SiO₂ von 1:2 bis 1:2,8. Als kristalline Silikate, die allein oder im Gemisch mit amorphen Silikaten vorliegen können, werden vorzugsweise kristalline Schichtsilikate der allgemeinen Formel Na₂SiₓO₂ₓ₊₁ • y H₂O eingesetzt, in der x, das sogenannte Modul, eine Zahl von 1,9 bis 4 und y eine Zahl von 0 bis 20 ist und bevorzugte Werte für x 2, 3 oder 4 sind. Bevorzugte kristalline Schichtsilikate sind solche, bei denen x in der genannten allgemeinen Formel die Werte 2 oder 3 annimmt. Insbesondere sind sowohl beta- als auch delta-Natriumdisilikate (Na₂Si₂O₅ • y H₂O) bevorzugt. Auch aus amorphen Alkalisilikaten hergestellte, praktisch wasserfreie kristalline Alkalisilikate der obengenannten allgemeinen Formel, in der x eine Zahl von 1,9 bis 2,1 bedeutet, können in erfindungsgemäßen Mitteln eingesetzt werden. In einer weiteren bevorzugten Ausführungsform erfindungsgemäßer Mittel wird ein kristallines Natriumschichtsilikat mit einem Modul von 2 bis 3 eingesetzt, wie es aus Sand und Soda hergestellt werden kann. Kristalline Natriumsilikate mit einem Modul im Bereich von 1,9 bis 3,5 werden in einer weiteren bevorzugten Ausführungsform erfindungsgemäßer Mittel eingesetzt. Falls als zusätzliche Buildersubstanz auch Alkalialumosilikat, insbesondere Zeolith, vorhanden ist, beträgt das Gewichtsverhältnis Alumosilikat zu Silikat, jeweils bezogen auf wasserfreie Aktivsubstanzen, vorzugsweise 1:10 bis 10:1. In Mitteln, die sowohl amorphe als auch kristalline Alkalisilikate enthalten, beträgt das Gewichtsverhältnis von amorphem Alkalisilikat zu kristallinem Alkalisilikat vorzugsweise 1:2 bis 2:1 und insbesondere 1:1 bis 2:1.

Buildersubstanzen sind, falls gewünscht, in den erfindungsgemäßen Mitteln vorzugsweise in Mengen bis zu 60 Gew.-%, insbesondere von 5 Gew.-% bis 40 Gew.-%, enthalten. Erfindungsgemäße Wäschenachbehandlungsmittel, wie z.B. Weichspüler, sind vorzugsweise frei von anorganischem Builder.

Als geeignete Persauerstoffverbindungen kommen insbesondere organische Persäuren beziehungsweise persaure Salze organischer Säuren, wie Phthalimidopercapronsäure, Perbenzoesäure oder Salze der Diperdodecandisäure, Wasserstoffperoxid und unter den Anwendungsbedingungen Wasserstoffperoxid abgebende anorganische Salze, wie Perborat, Percarbonat und/oder Persilikat, in Betracht. Sofern feste Persauerstoffverbindungen eingesetzt werden sollen, können diese in Form von Pulvern oder Granulaten verwendet werden, die auch in im Prinzip bekannter Weise umhüllt sein können. Besonders bevorzugt wird Alkalipercarbonat, Alkaliperborat-Monohydrat oder insbesondere in flüssigen Mitteln Wasserstoffperoxid in Form wässriger Lösungen, die 3 Gew.-% bis 10 Gew.-% Wasserstoffperoxid enthalten, ggf. eingesetzt. Falls ein erfindungsgemäßes Mittel Bleichmittel, wie vorzugsweise Persauerstoffverbindungen, enthält, sind diese in Mengen von vorzugsweise bis zu 50 Gew.-%, insbesondere von 5 Gew.-% bis 30 Gew.-%, vorhanden. Der Zusatz geringer Mengen bekannter Bleichmittelstabilisatoren wie beispielsweise von Phosphonaten, Boraten bzw. Metaboraten und Metasilikaten sowie Magnesiumsalzen wie Magnesiumsulfat kann zweckdienlich sein.

Als Bleichaktivatoren können Verbindungen, die unter Perhydrolysebedingungen aliphatische Peroxocarbonsäuren mit vorzugsweise 1 bis 10 Kohlenstoffatomen, insbesondere 2 bis 4 Kohlenstoffatomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxo-hexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat, 2,5-Diacetoxy-2,5-dihydrofuran und Enolester sowie acetyliertes Sorbitol und Mannitol beziehungsweise deren Mischungen (SORMAN), acylierte Zuckerderivate, insbesondere Pentaacetylglukose (PAG), Pentaacetylfruktose, Tetraacetylxylose und Octaacetyllactose sowie acetyliertes, gegebenenfalls N-alkyliertes Glucamin und Gluconolacton, und/oder N-acylierte Lactame, beispielsweise N-Benzoylcaprolactam. Hydrophil substituierte Acylacetale und Acyllactame werden ebenfalls bevorzugt eingesetzt. Auch Kombinationen konventioneller Bleichaktivatoren können eingesetzt werden. Derartige Bleichaktivatoren können im üblichen Mengenbereich, vorzugsweise in Mengen von 1 Gew.-% bis 10 Gew.-%, insbesondere 2 Gew.-% bis 8 Gew.-%, bezogen auf gesamtes Mittel, enthalten sein.

Zusätzlich zu den oben aufgeführten konventionellen Bleichaktivatoren oder an deren Stelle können auch Sulfonimine und/oder bleichverstärkende Übergangsmetallsalze beziehungsweise Übergangsmetallkomplexe als sogenannte Bleichkatalysatoren enthalten sein.

Als in den Mitteln verwendbare Enzyme kommen solche aus der Klasse der Proteasen, Cutinasen, Amylasen, Pullulanasen, Hemicellulasen, Cellulasen, Lipasen, Oxidasen und Peroxidasen sowie deren Gemische in Frage. Besonders geeignet sind aus Pilzen oder Bakterien, wie Bacillus subtilis, Bacillus licheniformis, Streptomyces griseus, Humicola lanuginosa, Humicola insolens, Pseudomonas pseudoalcaligenes oder Pseudomonas cepacia gewonnene enzymatische Wirkstoffe. Die gegebenenfalls verwendeten Enzyme können an Trägerstoffen adsorbiert und/oder in Hüllsubstanzen eingebettet sein, um sie gegen vorzeitige Inaktivierung zu schützen. Sie sind, falls gewünscht, in den erfindungsgemäßen Mitteln vorzugsweise in Mengen nicht über 5 Gew.-%, insbesondere von 0,2 Gew.-% bis 2 Gew.-%, enthalten.

Die Mittel können ggf. als optische Aufheller beispielsweise Derivate der Diaminostilbendisulfonsäure beziehungsweise deren Alkalimetallsalze enthalten. Geeignet sind zum Beispiel Salze der 4,4'-Bis(2-anilino-4-morpholino-1,3,5-triazinyl-6-amino)stilben-2,2'-disulfonsäure oder gleichartig aufgebaute Verbindungen, die anstelle der Morpholino-Gruppe eine Diethanolaminogruppe, eine Methylaminogruppe, eine Anilinogruppe oder eine 2-Methoxyethylaminogruppe tragen.

Zu den geeigneten Schauminhibitoren gehören beispielsweise Organopolysiloxane und deren Gemische mit mikrofeiner, gegebenenfalls silanierter Kieselsäure sowie Paraffinwachse und deren Gemische mit silanierter Kieselsäure oder Bisfettsäurealkylendiamiden. Mit Vorteilen werden auch Gemische aus verschiedenen Schaum-inhibitoren verwendet, zum Beispiel solche aus Silikonen, Paraffinen oder Wachsen. Vorzugsweise sind die Schauminhibitoren, insbesondere Silikon- und/oder Paraffin-haltige Schauminhibitoren, an eine granulare, in Wasser lösliche beziehungsweise dispergierbare Trägersubstanz gebunden. Insbesondere sind dabei Mischungen aus Paraffinwachsen und Bistearylethylendiamiden bevorzugt.

Zusätzlich können die Mittel auch Komponenten enthalten, welche die Öl- und Fettauswaschbarkeit aus Textilien positiv beeinflussen, sogenannte soil release-Wirkstoffe. Dieser Effekt wird besonders deutlich, wenn ein Textil verschmutzt wird, das bereits vorher mehrfach mit einem erfindungsgemäßen Mittel, das diese öl- und fettlösende Komponente enthält, gewaschen wurde. Zu den bevorzugten öl- und fettlösenden Komponenten zählen beispielsweise nichtionische Celluloseether wie Methylcellulose und Methylhydroxypropylcellulose mit einem Anteil an Methoxyl-Gruppen von 15 bis 30 Gew.-% und an Hydroxypropoxyl-Gruppen von 1 bis 15 Gew.-%, jeweils bezogen auf den nichtionischen Celluloseether, sowie die aus dem Stand der Technik bekannten Polymere der Phthalsäure und/oder der Terephthalsäure bzw. von deren Derivaten mit monomeren und/oder polymeren Diolen, insbesondere Polymere aus Ethylenterephthalaten und/oder Polyethylenglykolterephthalaten oder anionisch und/oder nichtionisch modifizierten Derivaten von diesen.

Die Mittel können auch Farbübertragungsinhibitoren, vorzugsweise in Mengen von 0,1 Gew.-% bis 2 Gew.-%, insbesondere 0,1 Gew.-% bis 1 Gew.-%, enthalten, die in einer bevorzugten Ausgestaltung der Erfindung Polymere aus Vinylpyrrolidon, Vinylimidazol, Vinylpyridin-N-Oxid oder Copolymere aus diesen sind.

Vergrauungsinhibitoren haben die Aufgabe, den von der Textilfaser abgelösten Schmutz in der Flotte suspendiert zu halten. Hierzu sind wasserlösliche Kolloide meist organischer Natur geeignet, beispielsweise Stärke, Leim, Gelatine, Salze von Ethercarbonsäuren oder Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich andere als die obengenannten Stärkederivate verwenden, zum Beispiel Aldehydstärken. Bevorzugt können Celluloseether, wie Carboxymethylcellulose (Na-Salz), Methylcellulose, Hydroxyalkylcellulose und Mischether, wie Methylhydroxyethylcellulose, Methylhydroxypropylcellulose, Methylcarboxymethylcellulose und deren Gemische, beispielsweise in Mengen von 0,1 bis 5 Gew.-%, bezogen auf die Mittel, eingesetzt werden.

Zu den in den erfindungsgemäßen Mitteln, insbesondere wenn sie in flüssiger oder pastöser Form vorliegen, verwendbaren organischen Lösungsmitteln gehören Alkohole mit 1 bis 4 Kohlenstoffatomen, insbesondere Methanol, Ethanol, Isopropanol und tert.-Butanol, Diole mit 2 bis 4 Kohlenstoffatomen, insbesondere Ethylenglykol und Propylenglykol, sowie deren Gemische und die aus den genannten Verbindungsklassen ableitbaren Ether. Derartige wassermischbare Lösungsmittel sind in den erfindungsgemäßen Mitteln vorzugsweise in Mengen von nicht über 30 Gew.-%, insbesondere von 6 Gew.-% bis 20 Gew.-%, vorhanden.

Zur Einstellung eines gewünschten, sich durch die Mischung der übrigen Komponenten nicht von selbst ergebenden pH-Werts können die erfindungsgemäßen Mittel system- und umweltverträgliche Säuren, insbesondere Citronensäure, Essigsäure, Weinsäure, Apfelsäure, Milchsäure, Glykolsäure, Bernsteinsäure, Glutarsäure und/oder Adipinsäure, aber auch Mineralsäuren, insbesondere Schwefelsäure, oder Basen, insbesondere Ammonium- oder Alkalihydroxide, enthalten. Derartige pH-Regulatoren sind optional in den erfindungsgemäßen Mitteln vorzugsweise nicht über 20 Gew.-%, insbesondere von 1,2 Gew.-% bis 17 Gew.-%, enthalten.

Die Herstellung fester erfindungsgemäßer Mittel (d.h. insbesondere Wasch- oder Reinigungsmittel) bereitet keine Schwierigkeiten und kann im Prinzip bekannter Weise, zum Beispiel durch Sprühtrocknen oder Granulation, erfolgen, wobei optionale Persauerstoffverbindung und optionaler Bleichkatalysator gegebenenfalls später zugesetzt werden. Zur Herstellung erfindungsgemäßer Mittel mit erhöhtem Schüttgewicht, insbesondere im Bereich von 650 g/L bis 950 g/L, ist ein einen Extrusionsschritt aufweisendes Verfahren bevorzugt. Die Herstellung flüssiger erfindungsgemäßer Mittel bereitet ebenfalls keine Schwierigkeiten und kann ebenfalls in bekannter Weise erfolgen.

Die Herstellung der erfindungsgemäßen Verbindungen der Formeln (II) bis (XXV) wird im Beispielteil exemplarisch anhand der Herstellung eines Citronellol- und Geraniol-enthaltenden Duftspeicherstoffes beschrieben. Über diese prinzipielle Syntheseroute sind die Verbindungen der Formeln (II) bis (XIII) darstellbar.

Gemäß einer bevorzugten Ausführungsform kann die erfindungsgemäße Lehre dazu eingesetzt werden, den Parfümanteil in Wasch-, Reinigungs- und Körperpflegemitteln signifikant herabzusetzen. Dadurch ist es möglich, parfümierte Produkte auch für solche besonders empfindlichen Konsumenten anzubieten, die normal parfümierte Produkte aufgrund spezieller Unverträglichkeiten und Irritationen nur eingeschränkt oder gar nicht verwenden können.

In verschiedenen Ausführungsformen der vorliegenden Erfindung liegen die Wasch- oder Reinigungsmittel in flüssiger oder in fester Form vor.

Ein bevorzugtes erfindungsgemäßes festes, insbesondere pulverförmiges Waschmittel kann neben der erfindungsgemäßen Verbindung insbesondere noch Komponenten enthalten, die z.B. ausgewählt sind aus den folgenden:
- Aniontenside, wie vorzugsweise Alkylbenzolsulfonat, Alkylsulfat, z.B. in Mengen von vorzugsweise 5 bis 30 Gew.-%
- Nichtionische Tenside, wie vorzugsweise Fettalkoholpolyglycolether, Alkylpolyglucosid, Fettsäureglucamid z.B. in Mengen von vorzugsweise 0,5 bis 15 Gew.-%
- Gerüststoffe, wie z.B. Zeolith, Polycarboxylat, Natriumcitrat, in Mengen von z.B. 0 bis 70 Gew.-%, vorteilhafterweise 5 bis 60 Gew.-%, vorzugsweise 10 bis 55 Gew.-%, insbesondere 15 bis 40 Gew.-%,
- Alkalien, wie z.B. Natriumcarbonat, in Mengen von z.B. 0 bis 35 Gew.-% vorteilhafterweise 1 bis 30 Gew.-%, vorzugsweise 2 bis 25 Gew.-%, insbesondere 5 bis 20 Gew.-%,
- Bleichmittel, wie z.B. Natriumperborat, Natriumpercarbonat, in Mengen von z.B. 0 bis 30 Gew.-% vorteilhafterweise 5 bis 25 Gew.-%, vorzugsweise 10 bis 20 Gew.-%,
- Korrosionsinhibitoren, z.B. Natriumsilicat, in Mengen von z.B. 0 bis 10 Gew.-%, vorteilhafterweise 1 bis 6 Gew.-%, vorzugsweise 2 bis 5 Gew.-%, insbesondere 3 bis 4 Gew.-%,
- Stabilisatoren, z.B. Phosphonate, vorteilhafterweise 0 bis 1 Gew.-%,
- Schauminhibitor, z.B. Seife, Siliconöle, Paraffine vorteilhafterweise 0 bis 4 Gew.-%, vorzugsweise 0,1 bis 3 Gew.-%, insbesondere 0,2 bis 1 Gew.-%,
- Enzyme, z.B. Proteasen, Amylasen, Cellulasen, Lipasen, vorteilhafterweise 0 bis 2 Gew.-%, vorzugsweise 0,2 bis 1 Gew.-%, insbesondere 0,3 bis 0,8 Gew.-%,
- Vergrauungsinhibitor, z.B. Carboxymethylcellulose, vorteilhafterweise 0 bis 1 Gew.-%,
- Verfärbungsinhibitor, z.B. Polyvinylpyrrolidon-Derivate, vorzugsweise 0 bis 2 Gew.-%,
- Stellmittel, z.B. Natriumsulfat, vorteilhafterweise 0 bis 20 Gew.-%,
- Optische Aufheller, z.B. Stilben-Derivat, Biphenyl-Derivat, vorteilhafterweise 0 bis 0,4 Gew.-%, insbesondere 0,1 bis 0,3 Gew.-%,
- ggf. weitere Duftstoffe
- ggf. Wasser
- ggf. Seife
- ggf. Bleichaktivatoren
- ggf. Cellulosederivate
- ggf. Schmutzabweiser,
Gew.-% jeweils bezogen auf das gesamte Mittel.

In einer anderen bevorzugten Ausführungsform der Erfindung liegt das Mittel in flüssiger Form vor, vorzugsweise in Gelform. Bevorzugte flüssige Wasch- oder Reinigungsmittel sowie Kosmetika haben Wassergehalte von z.B. 10 bis 95 Gew.-%, vorzugsweise 20 bis 80 Gew.-% und insbesondere 30 bis 70 Gew.-%, bezogen auf das gesamte Mittel. Im Falle von flüssigen Konzentraten kann der Wassergehalt auch besonders gering sein, z.B. < 30 Gew.-%, vorzugsweise < 20 Gew.-%, insbesondere < 15 Gew.-% betragen, Gew.-% jeweils bezogen auf das gesamte Mittel. Die flüssigen Mittel können auch nichtwässrige Lösungsmittel enthalten.

Ein bevorzugtes erfindungsgemäßes flüssiges, insbesondere gelförmiges Waschmittel kann neben der erfindungsgemäßen Verbindung insbesondere noch Komponenten enthalten, die z.B. ausgewählt sind aus den folgenden:
- Aniontenside, wie vorzugsweise Alkylbenzolsulfonat, Alkylsulfat, z.B. in Mengen von vorzugsweise 5 bis 40 Gew.-%
- Nichtionische Tenside, wie vorzugsweise Fettalkoholpolyglycolether, Alkylpolyglucosid, Fettsäureglucamid z.B. in Mengen von vorzugsweise 0,5 bis 25 Gew.-%
- Gerüststoffe, wie z.B. Zeolith, Polycarboxylat, Natriumcitrat, vorteilhafterweise 0 bis 15 Gew.-%, vorzugsweise 0,01 bis 10 Gew.-%, insbesondere 0,1 bis 5 Gew.-%,
- Schauminhibitor, z.B. Seife, Siliconöle, Paraffine, in Mengen von z.B. 0 bis 10 Gew.-%, vorteilhafterweise 0,1 bis 4 Gew.-%, vorzugsweise 0,2 bis 2 Gew.-%, insbesondere 1 bis 3 Gew.-%,
- Enzyme, z.B. Proteasen, Amylasen, Cellulasen, Lipasen, in Mengen von z.B. 0 bis 3 Gew.-%, vorteilhafterweise 0,1 bis 2 Gew.-%, vorzugsweise 0,2 bis 1 Gew.-%, insbesondere 0,3 bis 0,8 Gew.-%,
- Optische Aufheller, z.B. Stilben-Derivat, Biphenyl-Derivat, in Mengen von z.B. 0 bis 1 Gew.-%, vorteilhafterweise 0,1 bis 0,3 Gew.-%, insbesondere 0,1 bis 0,4 Gew.-%,
- ggf. weitere Duftstoffe
- ggf. Stabilisatoren,
- Wasser
- ggf. Seife, in Mengen von z.B. 0 bis 25 Gew.-%, vorteilhafterweise 1 bis 20 Gew.-%, vorzugsweise 2 bis 15 Gew.-%, insbesondere 5 bis 10 Gew.-%,
- ggf. Lösungsmittel (vorzugsweise Alkohole), vorteilhafterweise 0 bis 25 Gew.-%, vorzugsweise 1 bis 20 Gew.-%, insbesondere 2 bis 15 Gew.-%,
Gew.-% jeweils bezogen auf das gesamte Mittel.

Ein bevorzugter erfindungsgemäßer flüssiger Weichspüler kann neben dem erfindungsgemäßen Keton insbesondere noch Komponenten enthalten, die ausgewählt sind aus den folgenden:
- Kationische Tenside, wie insbesondere Esterquats, z.B. in Mengen von 5 bis 30 Gew.-%,
- Cotenside, wie z.B. Glycerolmonostearat, Stearinsäure, Fettalkohole, Fettalkoholethoxylate, z.B. in Mengen von 0 bis 5 Gew.-%, vorzugsweise 0,1 bis 4 Gew.-%,
- Emulgatoren, wie z.B. Fettaminethoxylate, z.B. in Mengen von 0 bis 4 Gew.-%, vorzugsweise 0,1 bis 3 Gew.-%,
- ggf. weitere Duftstoffe
- Farbstoffe, vorzugsweise im ppm-Bereich
- Stabilisatoren, vorzugsweise im ppm-Bereich
- Lösemittel, wie z.B. Wasser, in Mengen von vorzugsweise 60 bis 90 Gew.-%,
Gew.-% jeweils bezogen auf das gesamte Mittel.

Ferner ist ein Verfahren zur lang anhaltenden Beduftung von Oberflächen offenbart (nicht erfindungsgemäß), wobei ein erfindungsgemäße Verbindung gemäß Formeln (II) bis (XXV) oder ein erfindungsgemäßes Wasch- oder Reinigungsmittel, kosmetisches Mittel oder Luftpflegemittel auf die zu beduftende Oberfläche (z.B. Textil, Geschirr, Fußboden) aufgebracht wird und die genannte Oberfläche anschließend einer elektromagnetischen Strahlung mit Wellenlängen im Bereich von 200 bis 600 nm ausgesetzt wird.

### Beispiele

### Beispiel 1: Synthese Citral-Derivate

### Geranioxy-Acetonitril

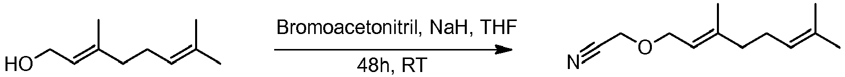

Zu einer Suspension aus Natriumhydrid (3.11 g, 77.8 mmol, 60% in Paraffinöl) in Tetrahydrofuran (100 mL) wird langsam Geraniol (10 g, 64.83 mmol) zugetropft. Die Reaktionslösung wird 2.5 h unter Rückfluss gerührt und anschließend auf Raumtemperatur abgekühlt. Es wird langsam Bromoacetonitril (5.43 mL, 77.8 mmol) über 1.5 h unter Eiskühlung zugetropft. Die dunkel braune Reaktionslösung wird anschließend 48 h bei Raumtemperatur gerührt. Die Reaktion wird durch die Zugabe von gesättigter, wässriger Ammoniumchlorid-Lösung beendet. Die Phasen werden getrennt, die wässrige Phase wird mit Diethylether (3 x 100 mL) extrahiert, die vereinten organischen Phasen mit gesättigter, wässriger Natriumchlorid-Lösung gewaschen, mit Natriumsulfat getrocknet, filtriert und das Lösungsmittel wird unter vermindertem Druck entfernt. Das Rohprodukt wird säulenchromatographisch gereinigt (PE:EE 10:1) und es wird das Geranioxy-Acetonitril (6.37 g, 32.96 mmol, 51 %) erhalten.

### 4-Methyl-Phenacyl-Geraniol

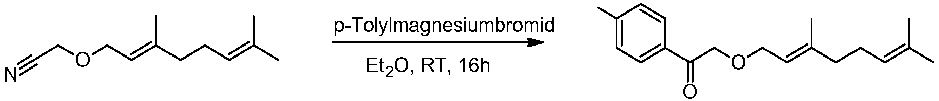

Geranioxy-Acetonitril (3.70 g, 19.14 mmol) wird in Diethylether (100 mL) gelöst. Anschließend wird unter Eiskühlung p-Tolylmagnesiumbromid (46 mL, 22.97 mmol, 0.5 M in Diethylether) über 10 Minuten zugetropft und die Reaktionslösung 16 h bei Raumtemperatur gerührt. Die Reaktion wird durch die Zugabe von 5 %-iger HCl (200 mL) beendet und die Mischung wird 2.5 h unter Rückfluss erhitzt. Anschließend wird auf Raumtemperatur abgekühlt, die Phasen werden getrennt und die wässrige Phase wird mit Dichlormethan (3 x 100 mL) extrahiert. Die vereinten organischen Phasen werden mit Magnesiumsulfat getrocknet, filtriert und das Lösungsmittel wird unter vermindertem Druck entfernt. Das Rohprodukt wird säulenchromatographisch gereinigt und es wird 4-Methyl-Phenayl-Geraniol (4.0 g, 14.0 mmol, 73 %) erhalten.

### Phenacyl-Geraniol

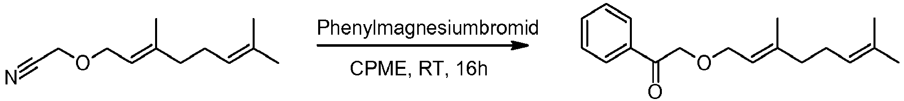

Geranioxy-Acetonitril (3.7 g, 19.14 mmol) wird in Cyclopentylmethylether (100 mL) gelöst. Anschließend wird unter Eiskühlung Phenylmagnesiumbromid (15 mL, 22.97 mmol, 1.6 M in Cyclopentylmethylether) über 10 Minuten zugetropft und die Reaktionslösung 16 h bei Raumtemperatur gerührt. Die Reaktion wird durch die Zugabe von 5 %-iger HCl (200 mL) beendet und die Mischung wird 2.5 h unter Rückfluss erhitzt. Anschließend wird auf Raumtemperatur abgekühlt, die Phasen getrennt und die wässrige Phase wird mit Diechlormethan (3 x 100 mL) extrahiert. Die vereinten organischen Phasen werden mit Magnesiumsulfat getrocknet, filtriert und das Lösungsmittel unter vermindertem Druck entfernt. Das Rohprodukt wird säulenchromatographisch gereinigt und es wird Phenacyl-Geraniol (3.65 g, 13.4 mmol, 69 %) erhalten.

### Beispiel 2: Synthese Citronellal-Derivate

### Citronelloxy-Acetonitril

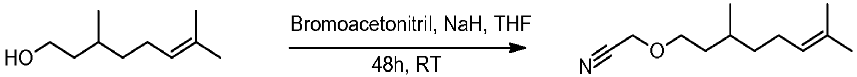

Zu einer Suspension aus Natriumhydrid (3.07 g, 76.79 mmol, 60 % in Paraffinöl) in Tetrahydrofuran (100 mL) wird langsam Citronellol (10 g, 63.99 mmol) zugetropft. Die Reaktionslösung wird 2.5 h unter Rückfluss gerührt und anschließend auf Raumtemperatur abgekühlt. Es wird langsam Bromoacetonitril (5.35 mL, 76.79 mmol) über 1.5 h unter Eiskühlung zugetropft. Die dunkel braune Reaktionslösung wird anschließend 48 h bei Raumtemperatur gerührt. Die Reaktion wird durch die Zugabe von gesättigter, wässriger Ammoniumchlorid-Lösung beendet. Die Phasen werden getrennt, die wässrige Phase wird mit Diethylether (3 x 100 mL) extrahiert, die vereinten organischen Phasen mit gesättigter, wässriger Natriumchlorid-Lösung gewaschen, mit Natriumsulfat getrocknet, filtriert und das Lösungsmittel wird unter vermindertem Druck entfernt. Das Rohprodukt wird säulenchromatographisch gereinigt (PE:EE 10:1) und es wird das Citronelloxy-Acetonitril (3.29 g, 16.85 mmol, 26 %) erhalten.

### 4-Methyl-Phenacyl-Citronellol

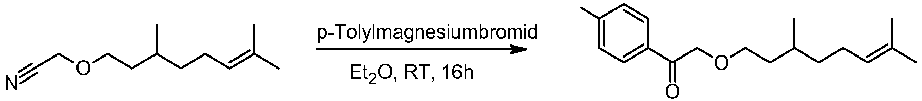

Citronelloxy-Acetonitril (2.81 g, 14.39 mmol) wird in Diethylether (100 mL) gelöst. Anschließend wird unter Eiskühlung p-Tolylmagnesiumbromid (34.54 mL, 17.27 mmol, 0.5 M in Diethylether) über 10 Minuten zugetropft und die Reaktionslösung 16 h bei Raumtemperatur gerührt. Die Reaktion wird durch die Zugabe von 5 %-iger HCl (200 mL) beendet und die Mischung wird 2.5 h unter Rückfluss erhitzt. Anschließend wird auf Raumtemperatur abgekühlt, die Phasen werden getrennt und die wässrige Phase wird mit Dichlormethan (3 x 100 mL) extrahiert. Die vereinten organischen Phasen werden mit Magnesiumsulfat getrocknet, filtriert und das Lösungsmittel wird unter vermindertem Druck entfernt. Das Rohprodukt wird säulenchromatographisch gereinigt und es wird 4-Methyl-Phenayl-Citronellol (2.22 g, 7.70 mmol, 53 %) erhalten.

### Phenacyl-Citronellol

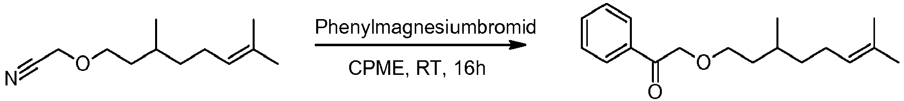

Citronelloxy-Acetonitril (3.29 g, 16.85 mmol) wird in Cyclopentylmethylether (100 mL) gelöst. Anschließend wird unter Eiskühlung Phenylmagnesiumbromid (12.63 mL, 20.21 mmol, 1.6 M in Cyclopentylmethylether) über 10 Minuten zugetropft und die Reaktionslösung 16 h bei Raumtemperatur gerührt. Die Reaktion wird durch die Zugabe von 5 %-iger HCl (200 mL) beendet und die Mischung wird 2.5 h unter Rückfluss erhitzt. Anschließend wird auf Raumtemperatur abgekühlt, die Phasen getrennt und die wässrige Phase wird mit Diechlormethan (3 x 100 mL) extrahiert. Die vereinten organischen Phasen werden mit Magnesiumsulfat getrocknet, filtriert und das Lösungsmittel unter vermindertem Druck entfernt. Das Rohprodukt wird säulenchromatographisch gereinigt und es wird Phenacyl-Citronellol (3.03 g, 11.0 mmol, 66 %) erhalten.

### Beispiel 3: Gelbfärbung

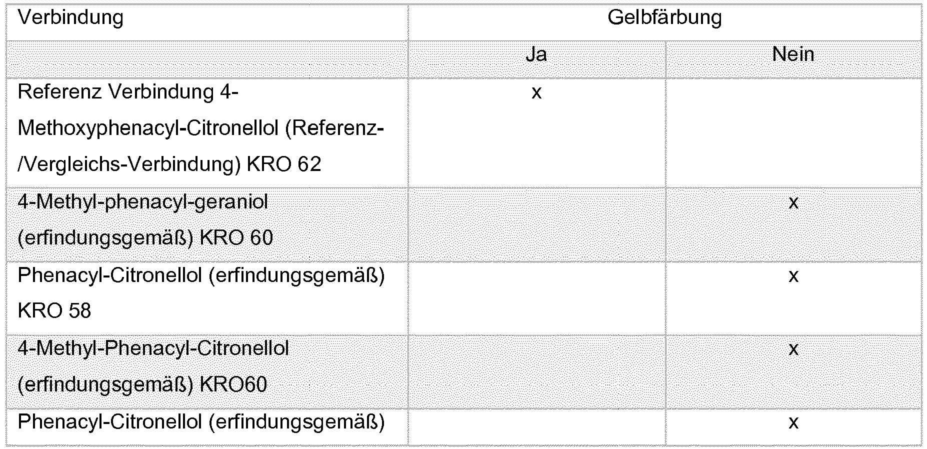

### Referenz Verbindung KRO 62

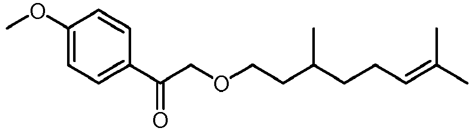

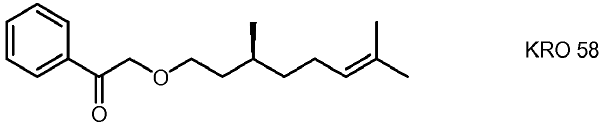

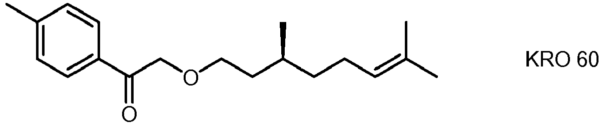

Fluoreszenzspektroskopische Messungen haben ferner ergeben, dass die Fluoreszenz bei der Verbindung KRO 62 bei 325 nm Anregung am intensivsten ist, was allerdings für die Duftstofffreisetzung nachteilig ist, da eine intensive Fluoreszenz gleichbedeutend mit einer nicht erfolgten Freisetzung des Duftstoffs ist. Somit sind die Verbindungen KRO 58 und KRO 60 auch im Hinblick auf die gewünschte Duftstofffreisetzung vorteilhaft, da die Anregung mit Licht der Wellenlänge 325 nm zum Bruch der Bindung und Freisetzung des Duftstoffs führt.

### Beispiel 4: Performance Duftstofffreisetzung

Die mit Vernel conc. Grundmasse und den Testsubstanzen on top gewaschenen Läppchen wurden 1 min lang mit Hilfe des Sonnenlichtsimulationsgerät Atlas Suntest XXL+ mit 0,58 W/cm² bestrahlt. Die Läppchen befanden sich dabei in einer Petrischale mit Quarzglasdeckel (durchlässig für das gesamte Lichtspektrum). Vor und nach der Bestrahlung wurde die Intensität des Duftes abgerochen und mit Werten auf einer Skala von 1 bis 10 (10 = intensiv, 0 = kein Geruch) bewertet. Die Intensität der Freisetzung der neu synthetisierten Substanzen 4-Methyl-Phenacyl-Geraniol und Phenacyl-Geraniol sowie 4-Methyl-Phenacyl-Citronellol und Phenacyl-Citronellol wurde mit den Duftstoffen Citral und Citronellal verglichen.

**Tabelle 4**

| Substanzen | Gehalt Riechstoff [%] | Dosierung [%] | Intensität vor Bestrahlung | Intensität nach Bestrahlung |
|---|---|---|---|---|
| Citronellal | 100 | 0,4 | 2 | 1 |
| Citral | 100 | 0,4 | 2 | 3 |
| 4-Methyl-Phenacyl-Geraniol (erfindungsgemäß) | 53,2 | 0,76 | 2 | 8 |
| 4-Methyl-Phenacyl-Citronellol (erfindungsgemäß) | 53,8 | 0,75 | 2 | 7 |
| Phenacyl-Citronellol (erfindungsgemäß) | 56,6 | 0,71 | 4-5 | 6-7 |
| 4-Methylphenacyl-methyl-geraniol (Formel (V)) | 51 | 0,78 | 5 | 8 |

Die Abspaltung des Duftstoffs unter Lichteinwirkung ist in folgendem Schema am Beispiel des 4-Methyl-Phenacyl-Geraniols und Phenacyl-Geraniols gezeigt, wobei sich als Spaltprodukte Citral und 1-(4-Methyl-phenyl)-ethanon bzw. 1-Phenylethanon ergeben:

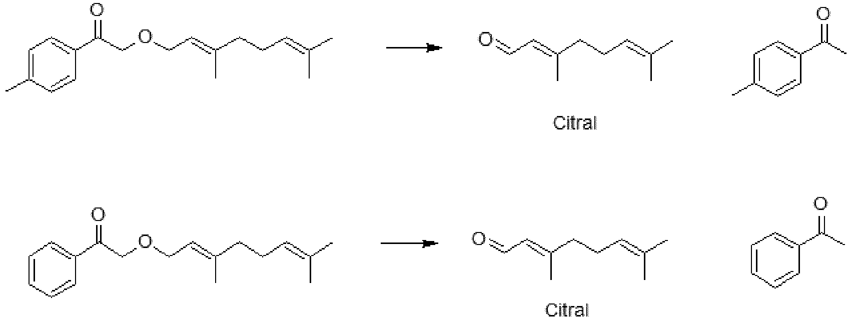

## Patentansprüche

1. Verbindung nach einer der folgenden Formeln (II) bis (XXV)

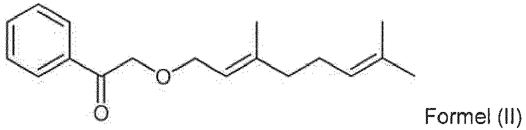

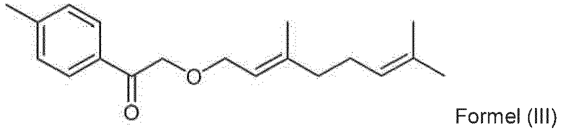

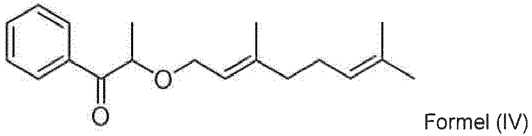

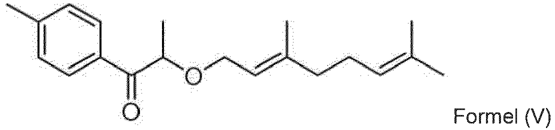

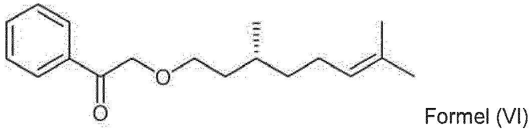

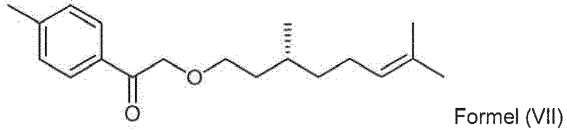

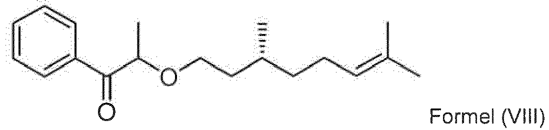

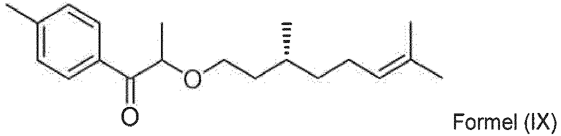

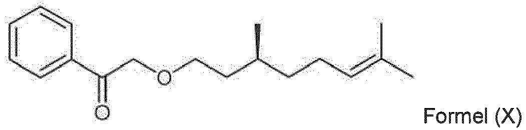

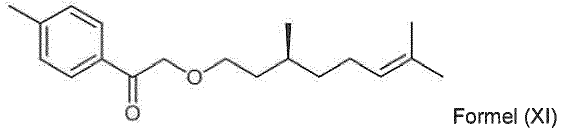

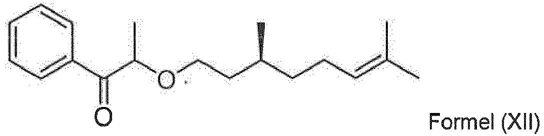

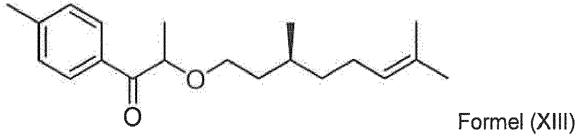

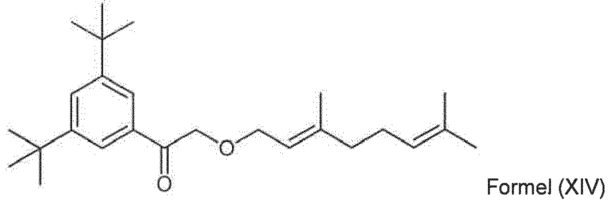

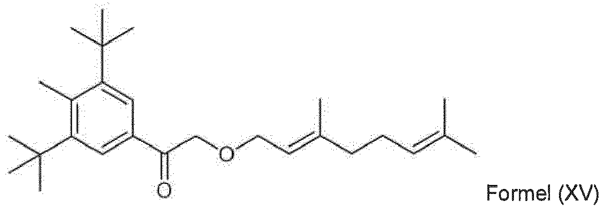

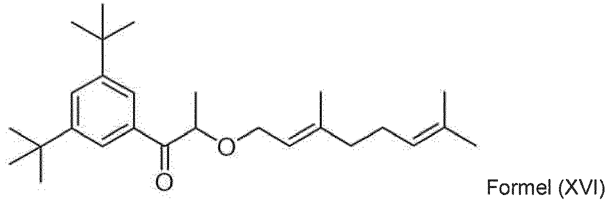

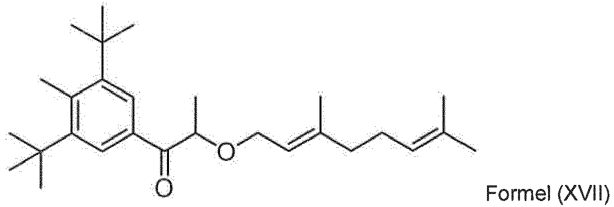

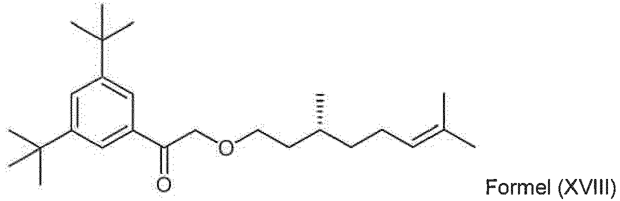

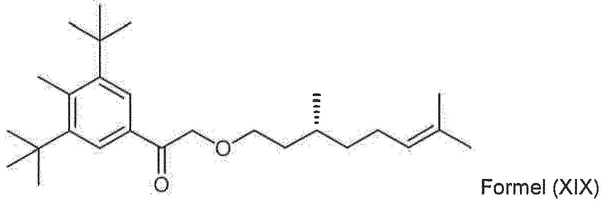

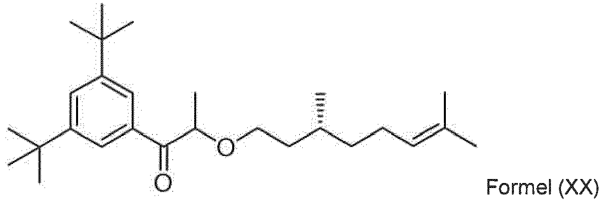

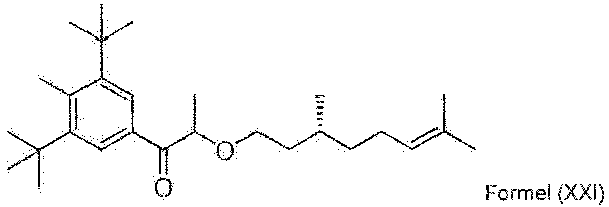

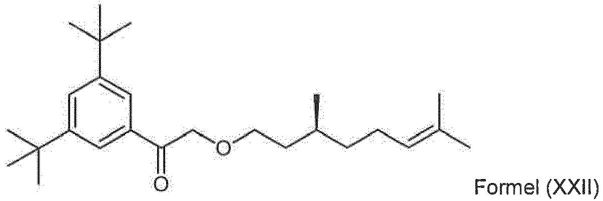

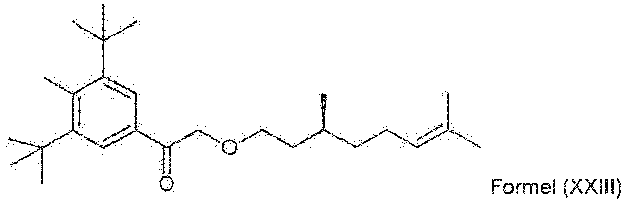

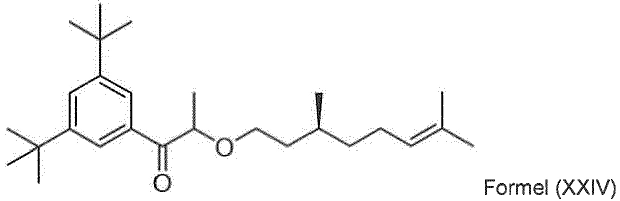

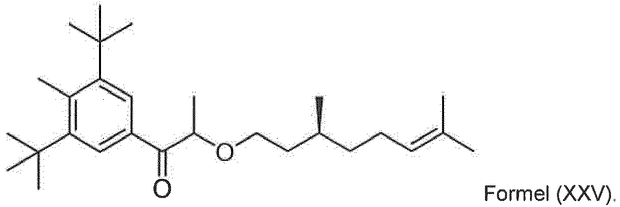

2. Wasch- oder Reinigungsmittel, enthaltend mindestens ein Verbindung nach Anspruch 1.

3. Wasch- oder Reinigungsmittel nach Anspruch 2, **dadurch gekennzeichnet, dass**
a. die mindestens eine Verbindung in Mengen zwischen 0,0001 und 5 Gew.-% bezogen auf das gesamte Mittel, enthalten ist, und/oder
b. es mindestens ein Tensid ausgewählt aus der Gruppe bestehend aus anionischen, kationischen, nichtionischen, zwitterionischen, amphoteren Tensiden und Mischungen daraus enthält, und/oder
c. es in flüssiger oder fester Form vorliegt.

4. Luftpflegemittel, enthaltend mindestens eine Verbindung nach Anspruch 1, **dadurch gekennzeichnet, dass** die genannte Verbindung in Mengen zwischen 0,0001 und 50 Gew.-% bezogen auf das gesamte Mittel, enthalten ist.

5. Kosmetisches Mittel, enthaltend mindestens eine Verbindung nach Anspruch 1, **dadurch gekennzeichnet, dass** die genannte Verbindung in Mengen zwischen 0,0001 und 50 Gew.-% bezogen auf das gesamte Mittel, enthalten ist.

## Claims

1. A compound according to one of the following formulas (II) to (XXV)

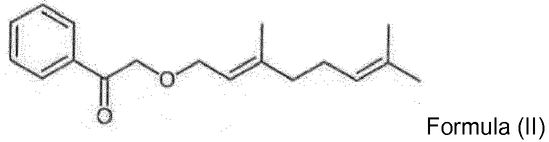

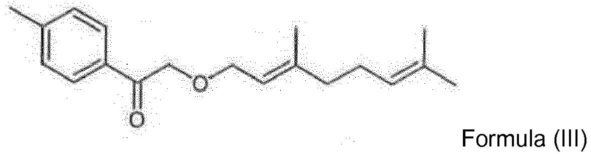

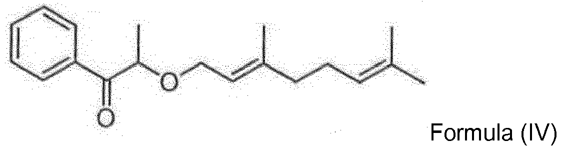

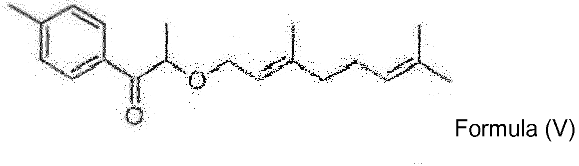

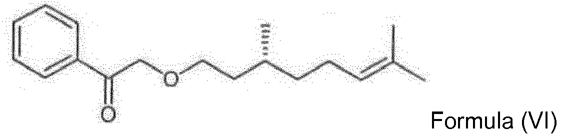

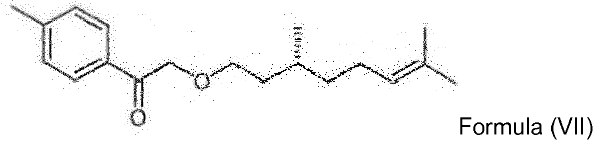

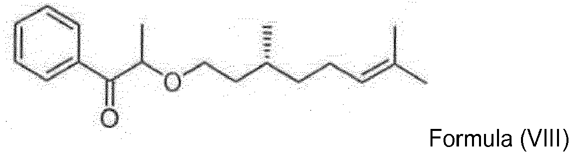

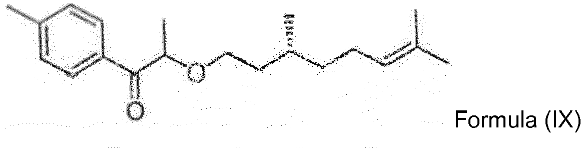

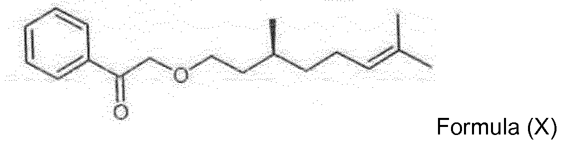

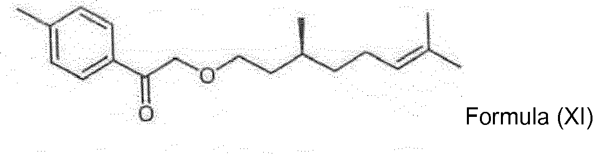

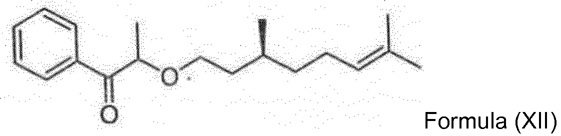

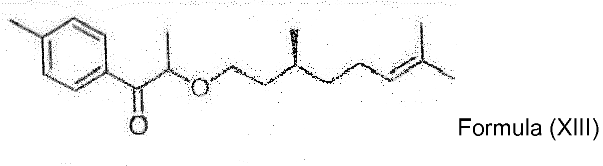

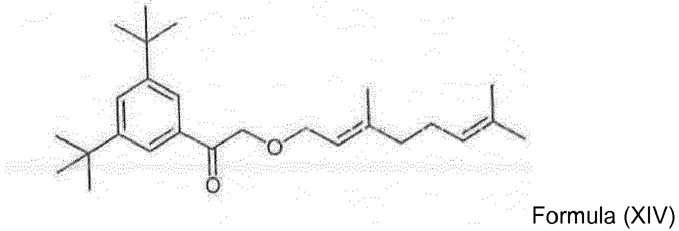

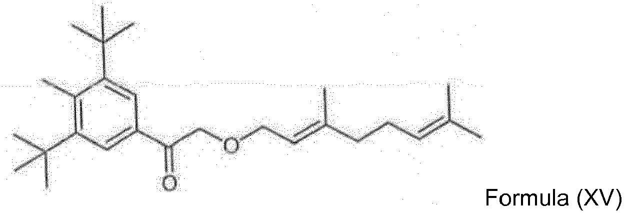

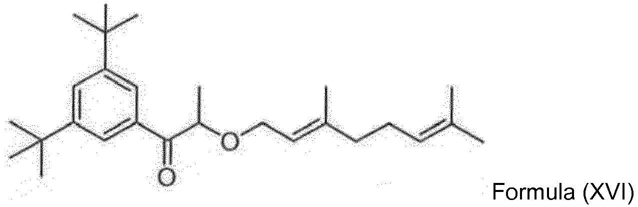

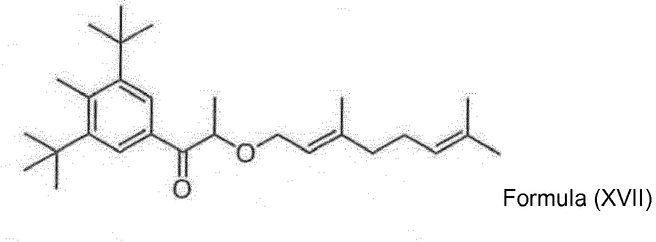

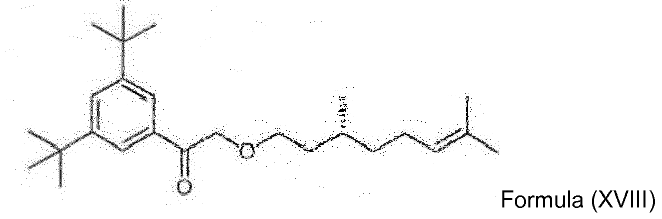

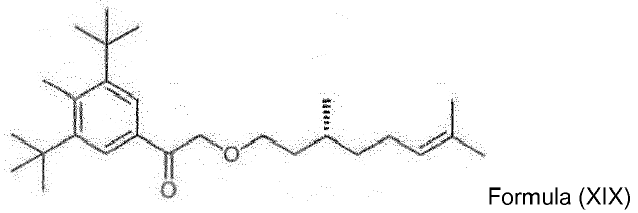

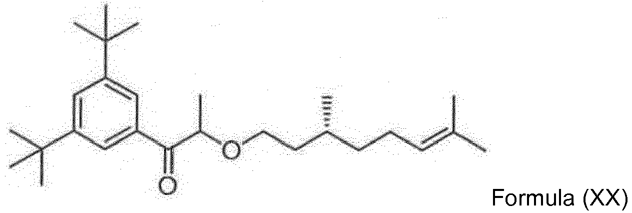

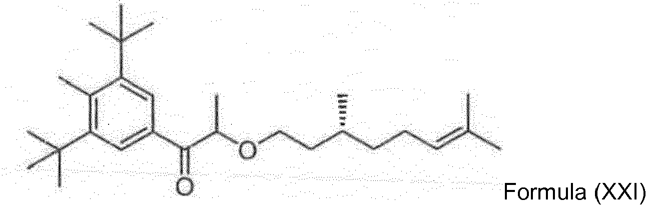

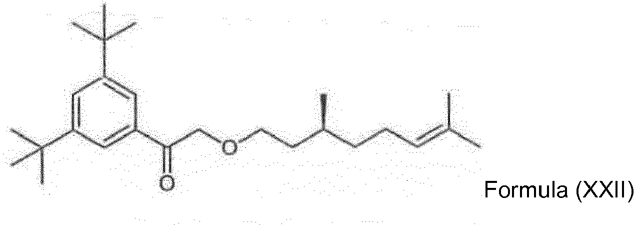

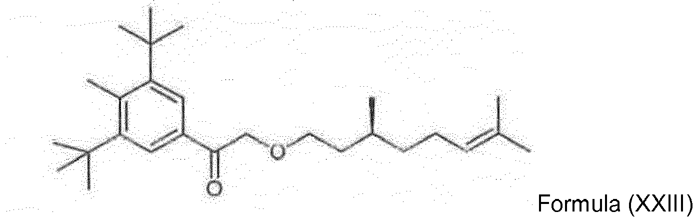

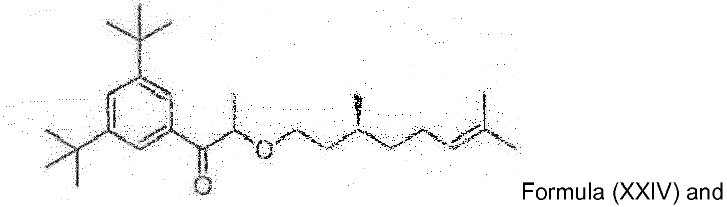

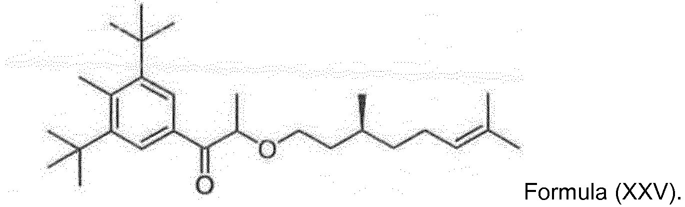

2. A washing or cleaning agent containing at least one compound according to claim 1.

3. The washing or cleaning agent according to claim 2, **characterized in that**
a. the at least one compound is contained in amounts between 0.0001 and 5 wt.% based on the total agent, and/or
b. it contains at least one surfactant selected from the group consisting of anionic, cationic, non-ionic, zwitterionic, amphoteric surfactants and mixtures thereof, and/or
c. it is present in liquid or solid form.

4. An air care agent containing at least one compound according to claim 1, **characterized in that** said compound is contained in amounts between 0.0001 and 50 wt.% based on the total agent.

5. A cosmetic agent containing at least one compound according to claim 1, **characterized in that** said compound is contained in amounts between 0.0001 and 50 wt.% based on the total agent.

## Revendications

1. Composé selon l'une des formules (II) à (XXV) suivantes

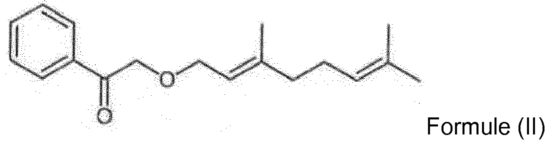

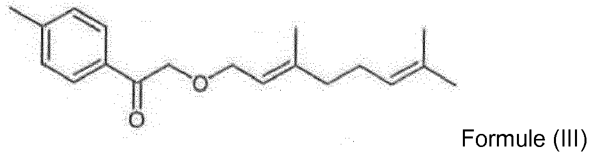

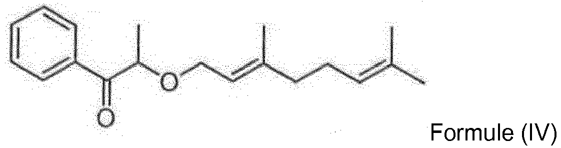

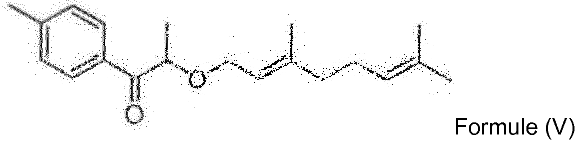

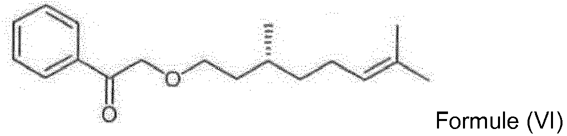

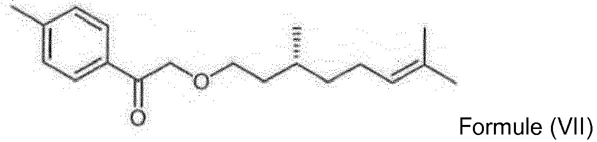

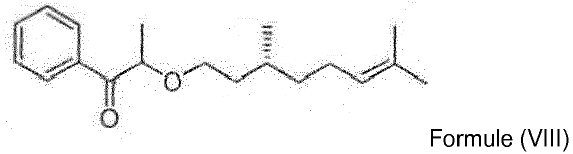

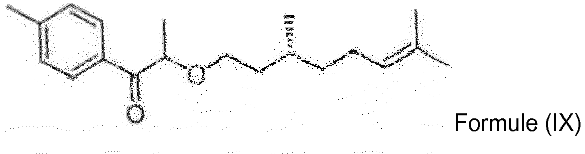

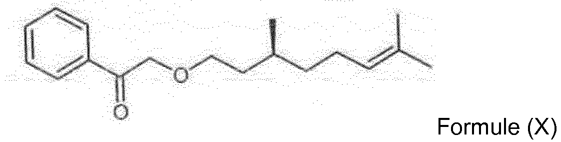

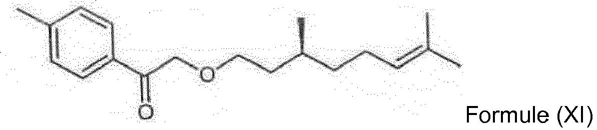

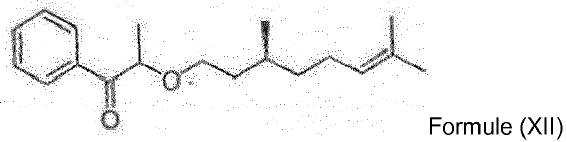

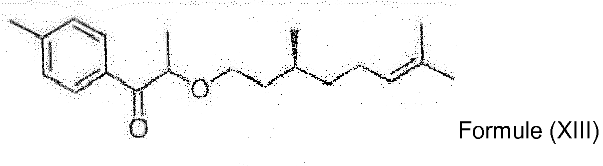

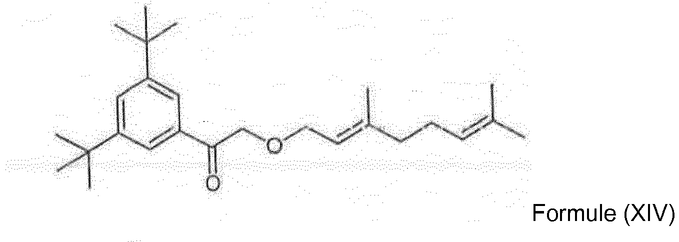

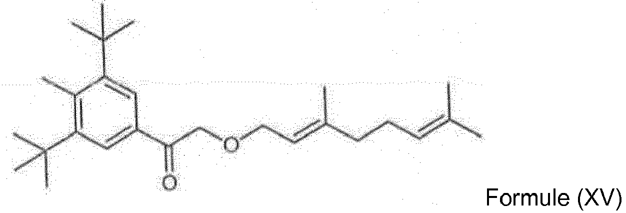

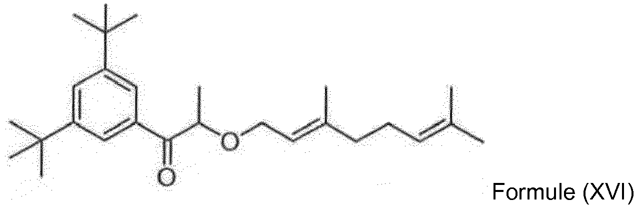

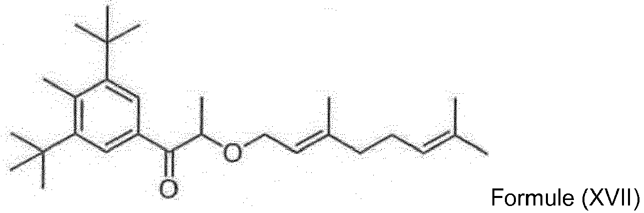

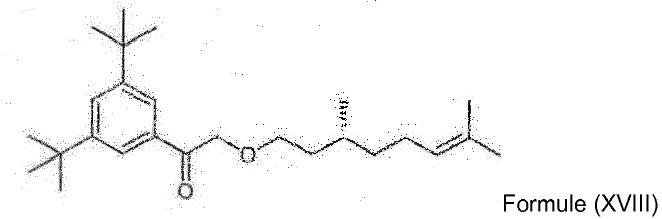

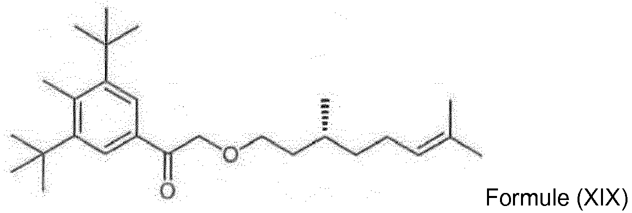

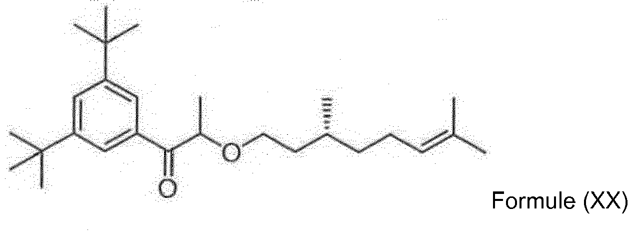

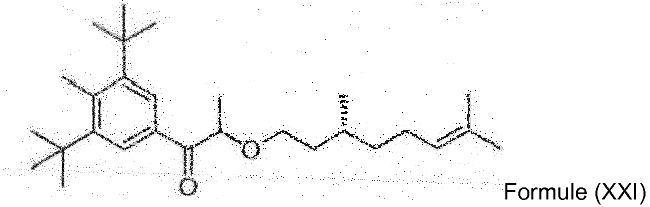

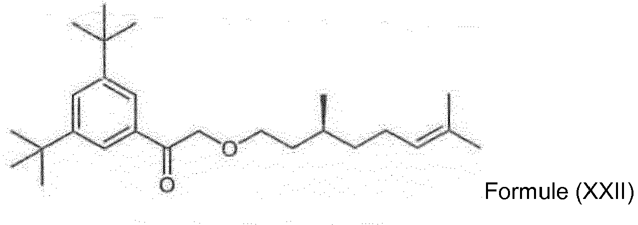

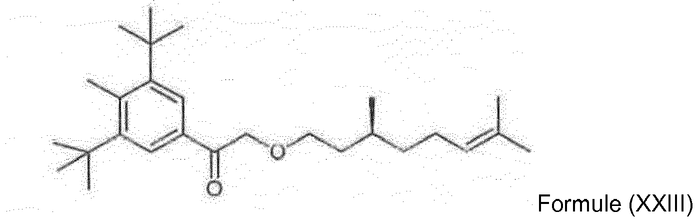

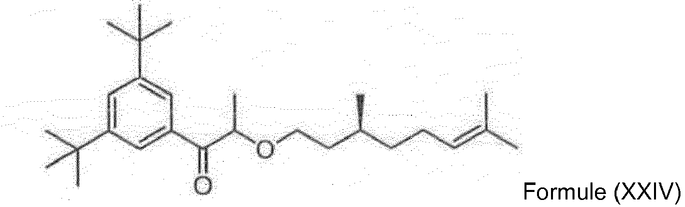

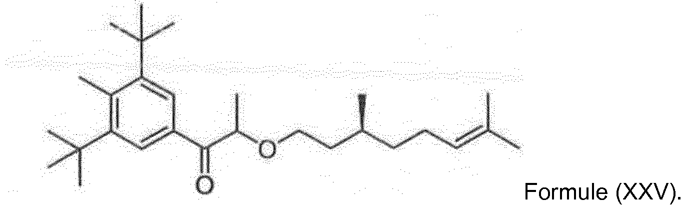

2. Détergent ou agent de nettoyage contenant au moins un composé selon la revendication 1.

3. Détergent ou agent de nettoyage selon la revendication 2, **caractérisé en ce**
a. **que** l'au moins un composé est présent en quantités comprises entre 0,0001 et 5 % en poids par rapport à l'agent total, et/ou
b. en ce qu'il contient au moins un tensioactif choisi dans le groupe constitué de tensioactifs anioniques, cationiques, non ioniques, zwitterioniques, amphotères et des mélanges de ceux-ci, et/ou
c. en ce qu'il se présente sous forme liquide ou solide.

4. Agent de traitement de l'air contenant au moins un composé selon la revendication 1, **caractérisé en ce que** ledit composé est présent en quantités comprises entre 0,0001 et 50 % en poids par rapport à l'agent total.

5. Agent cosmétique contenant au moins un composé selon la revendication 1, **caractérisé en ce que** ledit composé est présent en quantités comprises entre 0,0001 et 50 % en poids par rapport à l'agent total.
